# EUROPÄISCHE PATENTSCHRIFT

(11) **EP 2 595 612 B1**
(45) Veröffentlichungstag und Bekanntmachung des Hinweises auf die Patenterteilung: **11.06.2014**
(21) Anmeldenummer: 11738674.8
(22) Anmeldetag: 12.07.2011
(51) Int. Cl.: A61K 9/70

(54) **OPTIMIERTE HYDROGEL-MATRIXSYSTEME MIT EINEM GEHALT AN NICHTIONISCHEN EMULGATOREN**
OPTIMISED HYDROGEL MATRIX SYSTEMS CONTAINING NONIONIC EMULSIFIERS
SYSTÈME DE MATRICE D'HYDROGEL OPTIMISÉ PRÉSENTANT UNE CERTAINE TENEUR EN ÉMULSIFIANTS NONIONIQUES

(30) Priorität: 23.07.2010 DE 102010038312
(43) Veröffentlichungstag der Anmeldung: 29.05.2013
(73) Patentinhaber: Beiersdorf AG, 20253 Hamburg (DE)
(72) Erfinder: WÖLLER, Karl-Heinz, 20257 Hamburg (DE); URMANN, Katharina, 97753 Karlstadt (DE)
(86) Internationale Anmeldenummer: PCT/EP2011/061868
(87) Internationale Veröffentlichungsnummer: WO 2012/010465

(56) Entgegenhaltungen:
- WO-A1-2004/058315
- US-B1- 6 495 158

## Beschreibung

Die vorliegende Erfindung betrifft selbstklebende Polymermatrixsysteme enthaltend ein wässriges synthetisches Polymergel, bevorzugt ein Polyacrylsäurepolymergel, Meeresalgenextrakt und einen ein- oder mehrwertigen Alkohol, bei denen durch Zusatz nach Art und Menge an Emulgatoren Produkteigenschaften wie Oberflächenspannung und Klebkraft gezielt eingestellt werden können. Die Matrices können dabei mit hydrophilen oder auch hydrophoben Wirkstoffen dotiert werden, aber auch wirkstofffrei sein.

### Stand der Technik

Der Wirkmechanismus von erfindungsgemäßen Pflastern zur Verabreichung von Substanzen in die Haut ist analog dem der transdermalen therapeutischen Systeme.

Als transdermales Pflaster oder transdermales therapeutisches System (TTS oder TDS) wird ein Applikations-Medium für verschiedene Arzneiwirkstoffe bezeichnet, welches auf die Haut in Form eines Pflasters aufgeklebt wird. Auf diese Weise können Wirkstoffe direkt über die Haut in das Blutgefäßsystem gelangen, ohne den Magen-Darm-Trakt und die Leber passieren zu müssen.

Transdermale therapeutische Systeme zur Abgabe von kosmetischen, dermatologischen oder pharmazeutischen Wirkstoffen in bzw. durch die Haut sind seit langer Zeit bekannt.

Der Aufbau der transdermalen Pflaster ist wegen der unterschiedlichen Wirkstoffe verschieden. Dabei gibt es zwei Typen von transdermalen Pflastern. Bei den Matrix-Pflastern ist der Wirkstoff in einer aus einer oder mehreren Schichten bestehenden Matrix enthalten, die mit Hilfe einer Kleberschicht direkt auf der Haut aufliegt.

Bei den Membranpflastern liegt zwischen dem Wirkstoff-Reservoir und der Haut eine klebende Membran, die die kontrollierte Abgabe steuert. Unter einer Trägerfolie liegt ein Reservoir des Wirkstoffs, von dem dieser in die oberste Schicht der Haut, die Epidermis, abgegeben wird. Bei beiden Typen von Systemen diffundiert der Wirkstoff durch die Haut und gelangt über die hautnahen Blutgefäße in den Blutkreislauf.

Eine in der Fachliteratur gut beschriebene Ausführungsform solcher transdermalen Systeme stellen Matrixsysteme oder monolithische Systeme dar, in denen der Wirkstoff direkt in den druckempfindlichen Haftklebstoff eingearbeitet wird. Eine solche haftklebrige, wirkstoffhaltige Matrix ist üblicherweise im anwendungsfertigen Produkt auf der einen Seite mit einem für den Wirkstoff undurchlässigen Träger ausgestattet, auf der gegenüberliegenden Seite befindet sich eine mit einer Trennschicht ausgestatte Trägerfolie, die vor der Applikation auf die Haut entfernt wird (kleben&dichten, Nr. 42, 1998, S. 26 bis 30).

Monolithische selbstklebende Hydrogel-Matrixsysteme zur Verabreichung von pharmazeutischen, dermatologischen und/oder kosmetischen Wirkstoffen gehören in Asien, insbesondere in Japan, zu den traditionellen Anwendungen und werden im japanischen Arzneibuch unter dem Begriff "Cataplasma" (Breiumschlag) definiert. Cataplasmen werden danach gewöhnlich durch Mischen von Glycerin, Wasser oder anderer geeigneter flüssiger Substanzen mit fein pulverisierten Wirkstoffen unter Zusatz essentiellen Ölen zubereitet.

Nachteilig am derzeitigen Stand der Technik zu Cataplasmen ist, dass zur Herstellung der Basismatrices viele verschiedene Einzelkomponenten wie Gelbildner, Verdicker, Weichmacher, Feuchthaltemittel, Stabilisatoren, Emulgatoren, pH-Regulatoren, Antioxidantien etc. benötigt werden, bei wirkstoffhaltigen Cataplasmen evtl. noch zusätzlich Lösungsvermittler und Penetrationsbeschleuniger. Da sich Klebverhalten und Konsistenz einer solchen Matrix aus dem Zusammenwirken aller Einzelkomponenten ergeben, gestaltet sich eine gezielte Produktentwicklung/-optimierung hinsichtlich dieser grundlegenden Produktanforderungen entsprechend zeitaufwändig und schwierig.

Neuartige Systeme zur Herstellung von Hydrogel-Matrixsystemen bei denen die Produkteigenschaften durch Variation von nur vier Basisbestandteilen gezielt variiert werden können sind in DE 10260872 und DE 10260873 beschrieben. Nachteilig bei diesen selbstklebenden Hydrogel-Matrixsystemen ist, dass die Klebkraft zwar einstellbar ist, sich aber immer noch in dem für Cataplasmen/Hydrogel-Patches typischen sehr geringen Niveau befindet.

Pad (engl. Kissen, Belag), Patch (engl. Flicken) und Pflaster werden in diesen Anwendungsgebieten umgangssprachlich synonym verwendet. In der Kosmetik spricht man bei Pads von eher kleineren Produkten mit größerer Dicke, ggfs. auch mit einem etwas dickeren, also polsternden Träger, z. B. Eye Pads. Patches sind üblicherweise etwas großflächiger (z. B. 10 cm * 14 cm) und flacher, z. B. Anti-Cellulite Patches. Von Pflastern spricht man üblicherweise bei dermatologischen oder medizinischen Anwendungen, ohne Bezug auf Größe oder Ausstattung des Produkts.

### Aufgabe

Aufgabe der vorliegenden Erfindung ist es ein Hydrogel-Matrixsystem zu Verfügung zu stellen, welches für transdermales Pflaster geeignet ist und dessen Klebkraft gegenüber bekannten System erhöht ist und gezielt eingestellt werden kann.

### Lösung

Gelöst werden diese Aufgaben durch ein selbstklebendes Hydrogel-Matrixsystem enthaltend Wasser, mindestens eine Basiskomponente, mindestens einen Strukturbildner und mindestens ein nichtionisches Tensid, dadurch gekennzeichnet, dass das nichtionische Tensid einen HLB-Wert ≥ 14, insbesondere ≥ 16 aufweist und das Hydrokolloid-Matrixsystem eine Oberflächenenergie zwischen 25 und 40 mN/m, insbesondere zwischen 25 und 30 mN/m aufweist.

In den Unteransprüchen sind bevorzugte Ausführungsformen der Matrices offenbart. Die Erfindung umfasst darüber hinaus auch deren Verwendung.

Für den Fachmann überraschend wurde festgestellt, dass sich die Klebkraft durch Zusatz von nichtionischen Emulgatoren, in Abhängigkeit von deren HLB-Wert gezielt erhöhen lässt.

Ein erfindungsgemäßes Hydrogel-Matrixsystem weist daher zumindest die folgenden Bestandteile und Eigenschaften auf:
- Wasser
- Basiskomponente
- Strukturbildner und
- nichtionischer Emulgator,
wobei das Hydrokolloid-Matrixsystem eine Oberflächenenergie zwischen 25 und 40 mN/m, insbesondere zwischen 25 und 30 mN/m aufweist.

Gegebenenfalls weist ein erfindungsgemäßes Hydrogel-Matrixsystem noch weitere Basiskomponenten, Strukturbildner und/oder nichtionische Emulgatoren auf.

Die Hydrogel-Matrix befindet sich vorzugsweise auf einem flexiblen Träger, wie z. B. einer Folie oder einem Gewebe.

Besondere Ausführungsformen weisen einen kosmetischen, pharmazeutischen und/oder dermatologischen Wirkstoff auf, der in der Lage ist aus der Hydrogelmatrix in die Haut zu diffundieren.

### Basiskomponente

Erfindungsgemäß ist die Basiskomponente eine Polyacrylsäure, ein Polyacrylat (Polyacrylsäureester) oder ein Acrylsäureester-Copolymer, welche neben der Strukturausbildung in der Matrix auch das grundlegende Haftvermögen bestimmt.

Erfindungsgemäß vorteilhafte Polyacrylate sind Acrylat-Alkylacrylat-Copolymere, insbesondere solche, die aus der Gruppe der sogenannten Carbomere oder Carbopole (Carbopol® ist eine eingetragene Marke der B. F. Goodrich Company) gewählt werden. Insbesondere zeichnen sich das oder die erfindungsgemäß vorteilhaften Acrylat-Alkylacrylat-Copolymere durch die folgende Struktur aus:

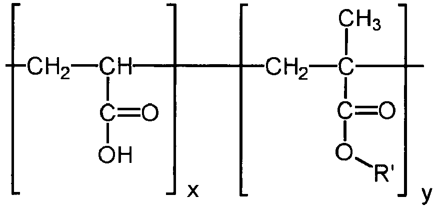

Darin stellen R' einen Alkylrest, insbesondere einen langkettigen Rest, und x und y Zahlen dar, welche den jeweiligen stöchiometrischen Anteil der jeweiligen Comonomere symbolisieren.

Erfindungsgemäß besonders bevorzugt sind Acrylat-Copolymere und/oder Acrylat-Alkylacrylat-Copolymere, welche unter den Handelbezeichnungen Carbopol® 1382, Carbopol® 981 und Carbopol® 5984 von der B. F.Goodrich Company erhältlich sind, bevorzugt Polyacrylate aus der Gruppe der Carbopole der Typen 980, 981, 1382, 2984, 5984 sowie besonders bevorzugt Carbomer 2001.

Ferner vorteilhaft sind Copolymere aus C₁₀₋₃₀-Alkylacrylaten und einem oder mehreren Monomeren der Acrylsäure, der Methacrylsäure oder deren Ester, die kreuzvernetzt sind mit einem Allylether der Saccharose oder einem Allylether des Pentaerythrit.

Als in Wasser gelbildendes Polymer eignet sich u.a. auch Affenbrotbaummehl.

Das in Wasser gelbildende Polymer, insbesondere Polyacrylsäure und/oder deren Copolymere, werden bevorzugt in einer Menge von 2 - 55 Gew.-%, besonders bevorzugt zwischen 5 - 30 Gew.% eingesetzt.

### Strukturbildner

In erfindungsgemäßen Hydrogel-Matrixsystemen ist Meeresalgenextrakt als Strukturbildner (verantwortlich für die Strukturfestigkeit) eingesetzt. Erfindungsgemäß besonders bevorzugt wird Agar-Agar, ein hydrophiles Kolloid von Polysaccharid-Struktur bestehend aus dem gelierenden Agarose und dem nichtgelierendem Agaropektin, das aus verschiedenen Meeresalgen der Rhodophyceen-Klasse durch Heißwasserextraktion, nachfolgendem Ausfrieren und anschließender Reinigung gewonnen wird. Industriell angeboten wird Agar-Agar z. B. von der Riedel de Haen AG.

Der Meeresalgenextrakt, insbesondere Agar-Agar oder Carrageenan, ein ebenfalls aus Algen gewonnenes Polysacharid, wird bevorzugt in einer Menge von 0,1 - 15 Gew.-%, besonders bevorzugt zwischen 0,5 - 5 Gew.-%, eingesetzt. Alle Prozentangaben beziehen sich dabei auf Gewichtsanteile der Polymermatrix sofern nicht Gegenteiliges angegeben ist.

Durch eine Erhöhung des Anteils an Meeresalgenextrakt in der Hydrogel-Matrix, wird die Festigkeit der Matrix erhöht. Dies erhöht jedoch auch die Steifigkeit und verringert die Klebrigkeit. Dieser Nachteil kann durch Zusatz von Alkohol, insbesondere von Glycerin, wieder ausgeglichen werden. Es kann somit vom Fachmann eine gewünschte Elastizität der resultierenden Hydrogel-Matrix bei konstantem Anteil an Meeresalgenextrakt eingestellt werden.

Grundlage für den Einsatz von Meeresalgenextrakt als Konsistenzfaktor ist, dass Meeresalgenextrakt, im Gegensatz zu insbesondere der weit verbreiteten Gelatine und anderen Konsistenzfaktoren, in Verbindung mit Alkoholen, wie z. B. Glycerin oder Propandiol, keine Gelbildung hervorruft.

Spezielle Ausführungsformen der Erfindung weisen als zweite Basiskomponente einen Anteil an ein oder mehrwertigen Alkoholen auf. Bevorzug sind kurzkettige Alkohole mit einer Kettenlänge von 2 bis 5 Kohlenstoffatomen, insbesondere Propandiol und Propantriol.

Da sich erfindungsgemäße ein- oder mehrwertige Alkohole, wie Glycerin oder Propandiol, in Wasser homogen verteilen, aber mit dem Meeresalgenextrakt keine Gele bilden, wirken solcher Art Alkohole somit als 'Elastizitätsfaktor' für die Matrices, denn die Kombination aus Meeresalgenextrakt und ein- oder mehrwertigen Alkoholen zeigt eine synergistische Wirkung in Bezug auf die Elastizität der Gelmatrices.

Ein- oder mehrwertige Alkohole wie z. B. Glycerin (1,2,3-Propantriol), sind unter anderem als Lösungsvermittler oder Feuchthaltemittel weit verbreitet eingesetzte Hilfsstoffe der pharmazeutischen Industrie.

Ein- oder mehrwertigen Alkohole, wie z. B. Glycerin, werden erfindungsgemäß bevorzugt in einer Menge von 1 - 85 Gew.-%, besonders bevorzugt zwischen 5 - 45 Gew.-% eingesetzt.

### Nichtionische Emulgatoren

Unter nichtionischen Emulgatoren im Sinne der Erfindung versteht man grenzflächenaktive Substanzen, die in wässriger Lösung keine Ionen bilden. Träger der grenzflächenaktiven Wirkung ist also das Gesamtmolekül. Die Hydrophilie solcher nichtionischer Emulgatoren wird durch den Anteil der polaren Gruppen im Molekül erreicht. Zu den nichtionischen Emulgatoren gehören Fettalkohole (Lauryl-, Cetyl-, Stearyl- oder Palmitylalkohol), partielle Fettsäureester mehrwertiger Alkohole mit gesättigten Fettsäuren (zum Beispiel Glycerolmonostearat, Pentaerythritolmonostearat, Ethylenglycolmonostearat, Propylenglycolmonostearat, Span oder Arlacel 20, 40 oder 60), partielle Fettsäureester mehrwertiger Alkohole mit ungesättigten Fettsäuren (z. B. Glycerolmonooleat, Pentaerythritolmonooleat, Span oder Arlacel 80, 85 oder C), ferner Polyoxyethylenester von Fettsäuren (z. B. Polyoxyethylenstearat), Polymerisationsprodukte aus Ethylenoxid und Propylenoxid an Fettalkoholen (Fettalkoholpolyglycolether) oder Fettsäuren (Fettsäureethoxylate, wie z.B. Cremophor A fest, Polyethylenglycole der Span Typen wie Tween 20, 40, 60, 61, 80, 81, 85 etc.).

### Beschreibung der Erfindung

Der Zusatz von Emulgatoren als Hilfsstoff zu Hydrogel-Matrices oder Cataplasmen an sich stellt dabei keine Neuerung dar. Emulgatoren werden vielfach als Komponente zugesetzt um bei einzelnen Matrices Löslichkeitseigenschaften für Wirk- und Hilfsstoffe zu optimieren.

Es war bislang nicht bekannt Emulgatoren in Abhängigkeit ihres HLB-Werts und ihrer Konzentration gezielt zur Erhöhung der Klebkraft für Hydrogel-Matrixsysteme einzusetzen.

Für den Fachmann überraschend ist, dass bei Hydrogel-Matrices eine lineare Abhängigkeit zwischen HLB-Wert und Einsatzkonzentration des Emulgators auf der einen Seite, sowie der daraus resultierenden Oberflächenenergie, der Klebkraft auf Stahl als auch der Wirkstofffreisetzung auf der anderen Seite besteht.

Die Klebkraft der erfindungsgemäßen Hydrogelpatches ist durch Zusatz eines Emulgators mit hohem HLB-Wert, einem HLB-Wert von ≥ 14, dabei gegenüber handelsüblichen Produkten auf analoger Matrixbasis signifikant höher.

Der HLB-Wert (von englisch hydrophilic-lipophilic balance, hydro-lipophiles Verhältnis) ist ein von Griffin (1950) eingeführtes Maß für die Wasser- bzw. Öllöslichkeit von vorwiegend nichtionischen Tensiden. Experimentell lässt sich der HLB-Wert z. B. durch die Phenol-Titrationsmethode bestimmen, indem man die Tensidlösung mit 5 %-iger Phenol-Lösung bis zur Trübe versetzt. Ferner kann der HLB-Wert (gas-)chromatographisch, durch Bestimmung der Dielektrizitätskonstanten oder kolorimetrisch ermittelt werden.

Für die Berechnung von HLB-Werten gilt: Für Fettsäureester mehrwertiger Alkohole gilt die Beziehung HLB = 20 · (1-VZ/SZ), wobei VZ für die Verseifungszahl und SZ für die Säurezahl des Esters stehen. Für Ethoxylate und deren Ester, bei denen die Verseifungszahl nur schwer zu bestimmen ist, gilt die Formel HLB = (E + P)/5, wobei E für die Zahl der Ethylenoxid-Einheiten und P für den Gehalt an mehrwertigen Alkoholen (Angabe in Gewichtsprozent) im Molekül stehen. Es sei darauf hingewiesen, dass diese Berechnungsmethode auf Polypropylenglycolether sowie anionische Tenside nicht angewandt werden kann.

Für eine Beurteilung der Anwendungsmöglichkeiten einer kosmetischen, dermatologischen oder pharmazeutischen Pflasterapplikation ist die Klebkraft ein wesentlicher Parameter.

Die Messung der Klebkraft von Matrixpflastern auf Stahl ist eine wichtige und preiswerte Methode um verschiedene Hydrogel-Matrices miteinander vergleichen zu können.

Zwar stimmen die absoluten Messwerte aus den Klebkraftmessungen auf Stahl zahlenmäßig nicht mit den Klebkräften auf der Haut überein, jedoch lässt sich auf die Klebkraft des Matrixsystems auf menschlicher Haut in Analogie schließen.

Die Herstellung der Polymermatrices erfolgt ohne Verwendung organischer Lösemittel, vorzugsweise bei 40 - 95°C, in handelsüblichen Mischern/Knetern oder kontinuierlich in geeigneten Extrudern.

Auf diese Weise lassen sich nur unter Verwendung von Wasser, in Wasser gelbildendem Polymer (Basiskomponente), Meeresalgenextrakt (Strukturbildner) und ein- oder mehrwertigem Alkohol als Ausgangsmaterialien gezielt weiche, geschmeidige, selbstklebende Hydrogelmatrices als Basis zur Herstellung und Anwendung als Pflaster, TTS, Cataplasmen oder kosmetischen Pads/Matrices herstellen.

Zum Vergleich unterschiedlicher Hydrogel-Matrixsysteme aus dem Stand der Technik und dem erfindungsgemäßen Hydrogel-Matrixsystem wurde die Klebkraft auf Stahl mittels automatischer Abzugsapparatur verschiedener Marktprodukte (Proben A bis G) gegenüber einer erfindungsgemäßen Matrix (Probe H) und einer im Stand der Technik (DE 10260872 und DE 10260873) beschriebenen Matrix (Probe I) gemessen.

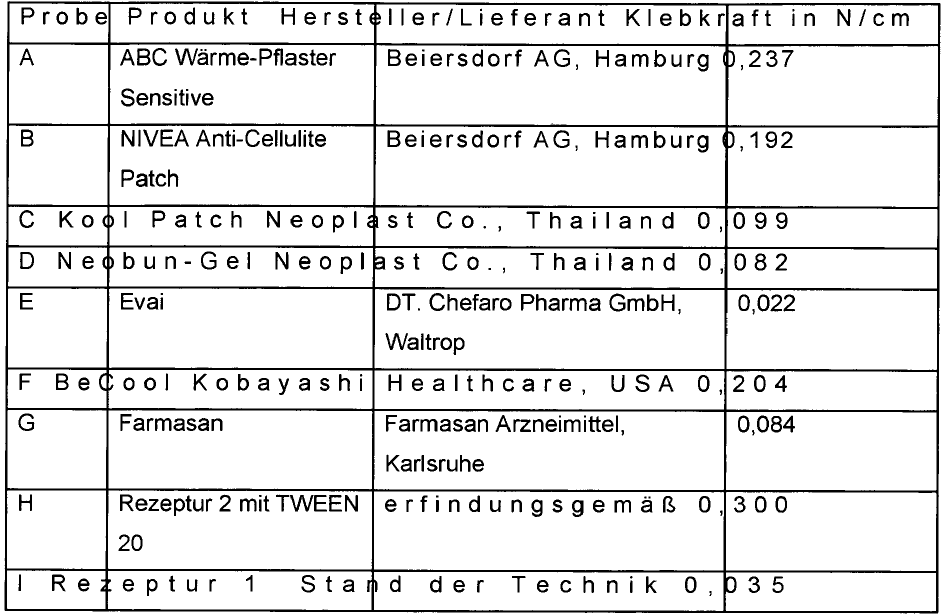

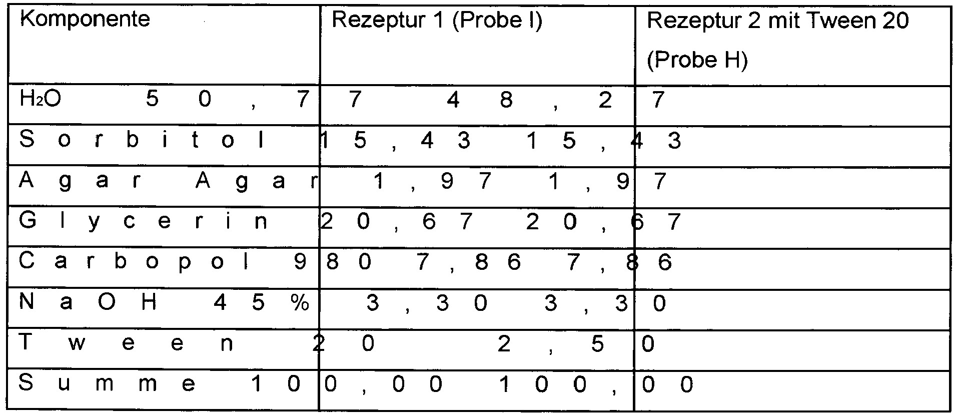

Alle Angaben in [Gew.-%]

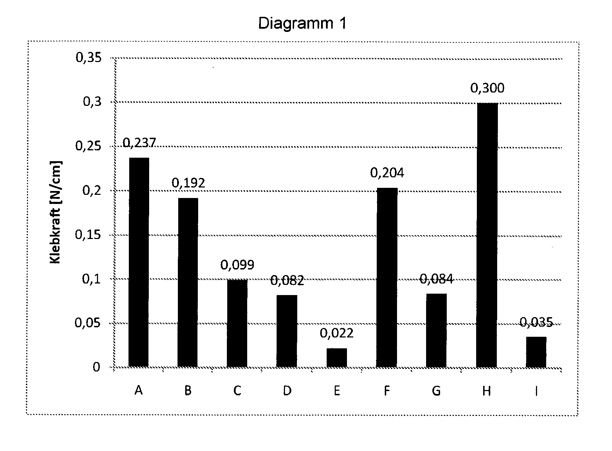

Das Ergebnis der Klebkraftmessungen ist in Diagramm 1 wiedergegeben. Die Klebkraft der Proben A bis I wurde wie unten beschrieben bestimmt.

Alle Vergleichsprodukte, A bis G, wiesen dabei eine Klebkraft von deutlich unter 0,3 N/cm auf. Die Probe I, ein Matrixsystem welches gegenüber der Erfindung keinen nichtionischer Emulgator aufweist, weist eine Klebkraft von 0,035 N/cm auf.

Die erfindungsgemäße Probe H, dessen Matrixsystem gegenüber dem Stand der Technik einen nichtionischen Emulgator enthält, weist mit einer Klebkraft von 0,300 N/cm, gegenüber dem Stand der Technik eine signifikant erhöhte Klebkraft auf.

Ein Zusammenhang zwischen den Eigenschaften eines Klebstoffs und seiner Klebkraft auf Haut ist bei der Oberflächenenergie gegeben. Es besteht die thermodynamische Voraussetzung, dass die gemessene Oberflächenenergie eines Klebstoffs, damit dieser gut klebt, kleiner oder gleich der von Haut sein muss.

Die Oberflächenenergie der menschlichen Haut lässt sich genauso messen wie die von flüssigem Klebstoff, oder wie in diesem Fall von selbstklebenden Matrixschichten. In der Literatur findet sich als Wert für die Oberflächenenergie sauberer, trockener menschlicher Haut 28 - 29 dyn cm-1 (entspricht mN/m). Dieser Wert variiert bei fettigeren Hautpartien und auch geringfügig zwischen den verschiedenen Individuen, kann aber als Richtwert angesehen werden. *Skin adhesives and skin adhesion,* S. Venkatraman and R. Gale (1998), Transdermal drug delivery systems in Biomaterials, Volume 19 S. 1119 - 1136*.*

Zur Klärung der grundlegenden Abhängigkeiten zwischen Emulgatorzusatz, dessen HLB-Wert, der daraus resultierenden Klebkräfte, Oberflächenenergien und Wirkstofffreisetzungen wurde die Basisrezeptur entsprechend DE 10260872 und DE 10260873 als Ausgangspunkt genommen und jeweils 2,5 Gew.-% an Wasser durch 2,5 Gew.-% Emulgator, wie in nachfolgender Rezeptur 2 wiedergegeben, ersetzt:

**Rezeptur 2:**

| Komponente | [Gew.-%] |
|---|---|
| H₂O | 48,27 |
| Sorbitol | 15,43 |
| Agar Agar | 1,97 |
| Glycerin | 20,67 |
| Carbopol 980 | 7,86 |
| NaOH 45% | 3,30 |
| Emulgator | 2,50 |
| Summe | 100,00 |

Folgende Tabelle gibt die Oberflächenenergien und Klebkräfte für Basisrezeptur 2 mit Emulgatoren, die einen unterschiedlichen HLB-Wert aufweisen, wieder:

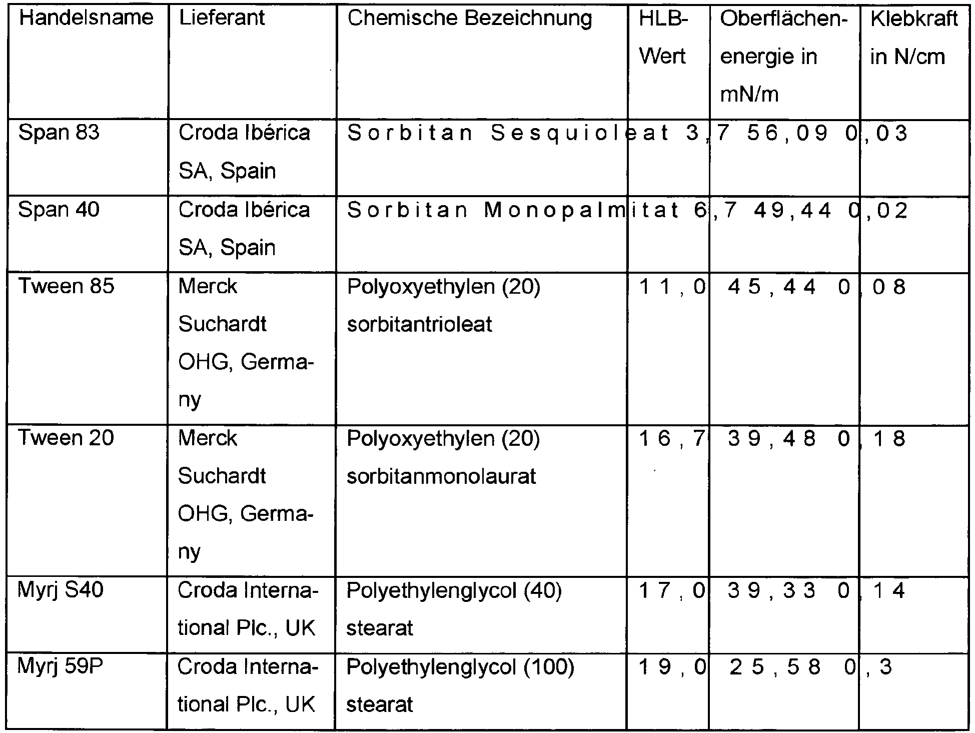

An den mit obigen Rezepturen hergestellten Hydrogel-Patches wurden die Klebkraft auf Stahl (angegeben in N/cm), die Oberflächenenergie (angegeben in mN/m) bestimmt.

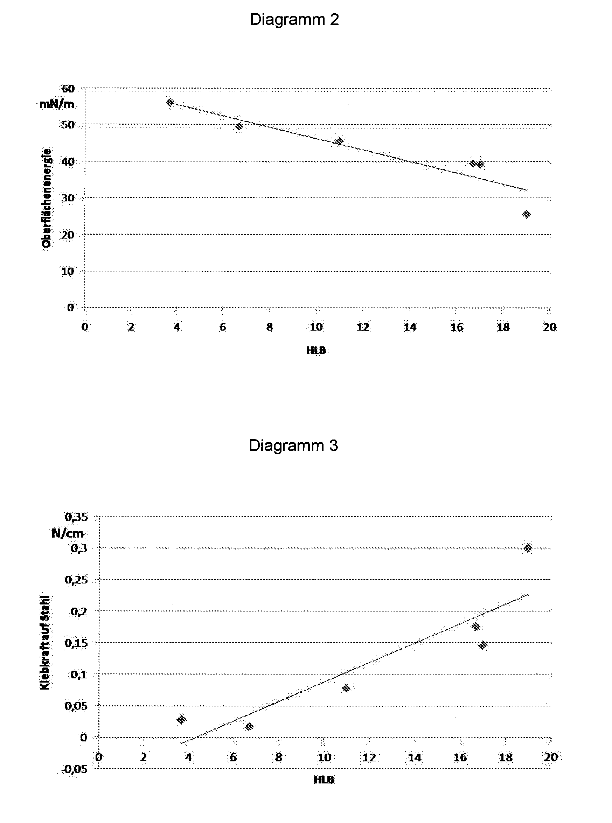

Diagramm 2 und Diagramm 3 zeigen die Abhängigkeit der Oberflächenenergie (Diagramm 2) und der Klebkraft (Diagramm 3) vom HLB-Wert des eingesetzten Emulgators.

Mit Erhöhung des HLB-Werts des zugesetzten Emulgators sinkt die Oberflächenenergie konstant ab.

Mit Erhöhung des HLB-Werts des zugesetzten Emulgators steigt die Klebkraft auf Stahl konstant an.

Es zeigt sich also, dass bei Zugabe einer konstanten Menge an Emulgatoren mit steigendem HLB-Wert auch die Klebkraft auf Stahl der resultierenden Hydrogel-Matrix linear erhöht wird, während gleichzeitig die Oberflächenenergie linear abnimmt.

Bei erfindungsgemäßen selbstklebenden Matrixsystemen auf Wasserbasis (Cataplasmen) kann die Klebkraft folglich gezielt erhöht bzw. eingestellt werden, in dem man über eine Kontaktwinkelmessung die Oberflächenenergie der Ausgangsmatrix bestimmt, diese in Beziehung zum Idealwert für eine Klebung auf Haut von ca. 28 - 29 mN/m setzt und durch Zugabe von Emulgator in Abhängigkeit von dessen HLB-Wert und Einsatzmenge annähert. Durch diese Vorgehensweise lässt sich die Klebkraft von wasserbasierten Matrixsystemen (Cataplasmen) gegenüber dem Stand der Technik mit einem erfindungsgemäßen System deutlich erhöhen.

Um den Einfluss des Emulgators auf die Klebkraft weiter zu verdeutlichen wurde die Rezeptur 2 als Ausgangspunkt genommen, mit jeweils 1,0 %, 2,5 % und 5,0 % des Emulgators Tween 20 (HLB-Wert 16,7) versetzt und vermessen. Aufgrund der starken Rezepturänderung von 1 % zu 5 % Emulgator und der damit verbundenen starken Änderung der Kohäsivität der Matrix konnte der Ausgleich dabei nicht ausschließlich gegen Wasser erfolgen, sondern musste auch zu geringen Anteilen gegen die Konsistenzfaktoren Glycerin und Polyacrylsäure erfolgen.

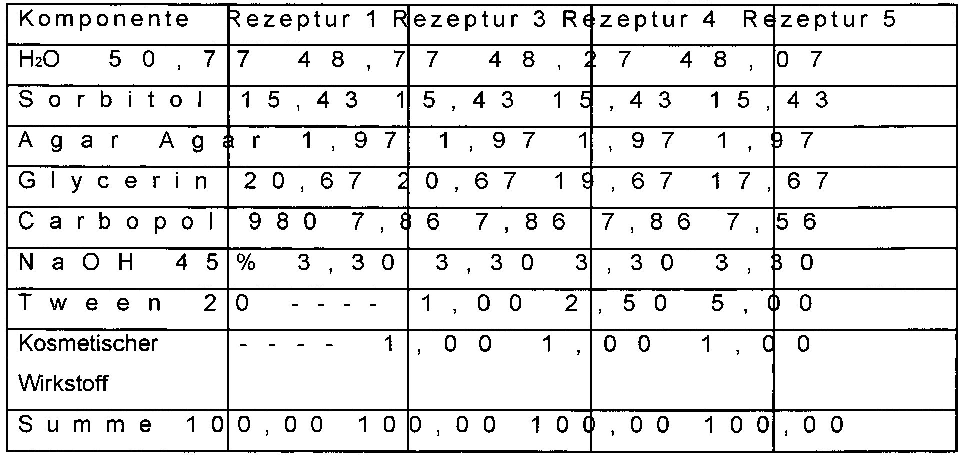

Alle Angaben in [Gew.-%]

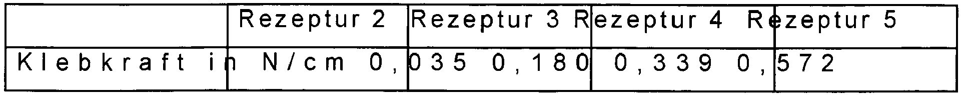

Zur Ausfertigung besonderer anwendungstechnischer Eigenschaften können die erfindungsgemäßen Hydrogel-Matrixsysteme mit entsprechenden Weichmachern, Lösungsvermittlern, Penetrationsenhancern, Neutralisationsmitteln wie z. B. Tromethamol (2-Amino-2-(hydroxymethyl)-1,3-propandiol), Triethanolamin (2,2',2"-Nitrilotriethanol) oder NaOH, Füllstoffen und/oder anderen bekannten Zusätzen versetzt werden, deren Zusatz jedoch nicht zwingend ist.

### Wirkstoffe

In einer erfindungsgemäß besonders bevorzugten Ausführungsform enthält das Hydrogel-Matrixsystem dermatologische und/oder kosmetische Wirkstoffe zur kontrollierten lokalen bzw. systemischen Abgabe an/in die Haut, in Mengen von 0 - 35 Gew.-%, bevorzugt 0 - 15 Gew.-%, ganz besonders bevorzugt 0,02 - 2 % enthalten.

Bei der Einarbeitung hydrophober Wirkstoffe kann es von Nutzen sein, Cyclodextrine zur Verkapselung einzusetzen.

Da es sich bei der erfindungsgemäßen Hydrogel-Matrix um eine wasserhaltige Applikationsform handelt, erreicht man zusätzlich einen kühlenden Effekt, der *per se* schon kosmetisch angenehm ist und zum Wohlbefinden beiträgt. Diese positive Wirkung kann durch die Zugabe weiterer pflegender Bestandteile verstärkt werden. Neben Glycerin können insbesondere Serinol (3-Amino-1,2-Propandiol) bzw. Isoserinol (2-Amino-1,3-Propandiol) sowie Harnstoff und PCA (Pyrrolidoncarbonsäure) als feuchtigkeitsspendende Substanzen beigefügt werden. Selbstverständlich können auch weitere Substanzen zu diesem Zwecke beigefügt werden.

Als besonders geeignete Wirkstoffe im Sinne der Erfindung können den genannten kosmetischen Matrices/Pads Wirkstoffe entweder einzeln oder auch in Kombination beigefügt werden.

Bei der vorliegenden Erfindung hat sich völlig überraschenderweise gezeigt, dass die erfindungsgemäßen Formulierungen sich auch ganz besonders eignen für den Einsatz von Wirkstoffen, die den Zustand der Haut positiv beeinflussen. So zeigte sich, dass Wirkstoffe zur positiven Beeinflussung der Altershaut, die die Entstehung von Falten oder auch bestehenden Falten vermindern. Als besonders bevorzugte Wirkstoffe gelten daher Biochinone, insbesondere Ubichinon Q10, Kreatin, Kreatinin, Carnitin, Acetylcarnitin, Biotin, Isoflavon und Isoflavonoide, Genistein, Arctiin, Cardiolipin, Liponsäure, Anti Freezing Proteine, Hopfen- und Hopfen-Malz-Extrakte, und/oder die Restrukturierung des Bindegewebes fördernde Stoffe, Isoflavonoide sowie Isoflavonoid-haltige Pflanzenextrakte wie z. B. Soja- und Klee-Extrakte, die in den erfindungsgemäßen Matrices sehr gut verwendet werden können. Auch zeigte sich, dass sich die Matrix in besonderer Weise eignet, Wirkstoffe zur Unterstützung der Hautfunktionen bei trockener Haut, wie beispielsweise Vitamin C, Biotin, Carnitin, Kreatin, Kreatinin, Propionsäure, Glycerin, Grüntee-Extrakte, Eucalyptusöl, Harnstoff und Mineralsalze wie z. B. NaCl, Meeresmineralien sowie Osmolyte wie z. B. Taurin, Inositol, Betain, quartäre Ammoniumverbindungen, zu verwenden. In ähnlicher Weise erwies sich die Einarbeitung von Wirkstoffen zur Linderung bzw. positiven Beeinflussung von, irritativen Hautzuständen, sei es bei empfindlicher Haut im allgemeinen oder bei durch Noxen gereizter Haut (UV-Licht, Chemikalien), als vorteilhaft. Hier sind Wirkstoffe zu nennen wie Sericoside, verschiedene Extrakte des Süssholzes, Licochalcone A, Silymarin bzw. Silyphos, Dexpanthenol, Ethanol, Inhibitoren des Prostaglandinstoffwechsels, insbesondere der Cyclooxygenase, und des Leukotrienstoffwechsels, insbesondere der 5-Lipoxygenase, aber auch des 5-Lipoxygenase Inhibitor Proteins, FLAP. Auch erwies sich die Einarbeitung von Modulatoren der Pigmentierung als vorteilhaft. Hier sind Wirkstoffe zu nennen, die die Pigmentierung der Haut vermindern und so zu einer kosmetisch gewünschten Aufhellung der Haut führen und/oder das Auftreten von Altersflecken reduzieren und/oder bestehende Altersflecken aufhellen, wie Tyrosinsulfat, Dioic acid (8-Hexadecen-1,16-dicarbonsäure), Liponsäure und Liponamid, verschiedene Extrakte des Süssholzes, Kojisäure, Hydrochinon, Arbutin, Fruchtsäuren, insbesondere Alpha-Hydroxy-Säuren (AHAs), Bearberry (Uvae ursi), Ursolsäure, Ascorbinsäure, Grüntee-Extrakte, Aminoguanidin und/oder Pyridoxamin. In gleicher Weise erwiesen sich die erfindungsgemäßen Matrices als hervorragende Grundlage für Wirkstoffe, die eine verstärkte/schnellere Bräunung der Haut herbeiführen (Advanced Glycation Endproducts (AGE), Lipofuscine, Nukleinsäure-Oligonukleotide, Purine und Pyrimidine, NOfreisetzende Substanzen, sei es mit oder ohne Einfluss von UV-Licht.

Zur Prophylaxe vor oxydativen und degenerativen Schäden und insbesondere zur Behandlung von denselben hat es sich als überraschenderweise als sinnvoll erwiesen, den kosmetischen Matrices/Pads Antioxidantien hinzuzufügen. Vorteilhaft werden die Antioxidantien gewählt aus der Gruppe bestehend aus Aminosäuren, z. B. Glycin, Lysin, Arginin, Cystein, Histidin, Tyrosin, Tryptophan, und deren Derivate (als Salz-, Ester-, Ether-, Zucker-, Nukleotid-, Nukleosid-, Peptid- und Lipid-Verbidung), Imidazole, z. B. Urocaninsäure, und deren Derivate, als Salz-, Ester-, Ether-, Zucker-, Nukleotid-, Nukleosid-, Peptid- und/oder Lipid-Verbidung, Peptide wie D,L-Carnosin, D-Carnosin, L-Carnosin, Anserin und deren Derivate, z.B. als Salz-, Ester-, Ether-, Zucker-, Thiol-, Nukleotid-, Nukleosid-, Peptid- und Lipid-Verbidung, Carotinoide, Carotine, z. B. α-Carotin, β-Carotin, ψ-Lycopin, Phytoen, und deren Derivate, z. B. als Salz-, Ester-, Ether-, Zucker-, Nukleotid-, Nukleosid-, Peptid- und/oder Lipid-Verbidung, Chlorogensäure und deren Derivate, als Salz-, Ester-, Ether-, Zucker-, Thiol-, Nukleotid-, Nukleosid-, Peptid- und/oder Lipid-Verbidung, Aurothioglucose, Propylthiouracil und andere Thiole, z.B. Thioredoxin, Liponsäure, Glutathion, Cystein, Cystin, Cystamin und deren Glycosyl-, N-Acetyl-, Methyl-, Ethyl-, Propyl-, Amyl-, Butyl- und Lauryl-, Palmitoyl-, Oleyl-, γ-Linoleyl-, Cholesteryl- und Glycerylester, sowie deren Salze, Dilaurylthiodipropionat, Distearylthiodipropionat, Thiodipropionsäure und deren Derivate, als Salz-, Ester-, Ether-, Zucker-, Thiol-, Nukleotid-, Nukleosid-, Peptid- und/oder Lipid-Verbidung, sowie Sulfoximinverbindungen, z.B. Homocysteinsulfoximin, Buthioninsulfone, Penta-, Hexa-, Heptathioninsulfoximin, in sehr geringen verträglichen Dosierungen, z.B. pmol bis µmol/kg. Ferner (Metall)-Chelatoren, z. B. Apoferritin, Desferral, Lactoferrin, α-Hydroxyfettsäuren, Palmitinsäure, Phytinsäure, und deren Derivate, als Salz-, Ester-, Ether-, Zucker-, Thiol-, Nukleotid-, Nukleosid-, Peptid- und/oder Lipid-Verbidung, α-Hydroxysäuren, z.B. Citronensäure, Milchsäure, Apfelsäure, Huminsäure, Gallensäure, Gallenextrakte, Bilirubin, Biliverdin, Melanin, EDTA, EGTA und deren Derivate, ungesättigte Fettsäuren und deren Derivate, z. B. γ-Linolensäure, Linolsäure, Ölsäure, Folsäure und deren Derivate, Furfurylidensorbitol und dessen Derivate, Ubichinon, Ubichinol, Plastochinon und deren Derivate, als Salz-, Ester-, Ether-, Zucker-, Thiol-, Nukleotid-, Nukleosid-, Peptid- und Lipid-Verbidung, Vitamin C und Derivate, z.B. Ascorbylpalmitat, Mg-Ascorbylphosphat, Ascorbylacetat, Tocopherole und Derivate, z. B. Vitamin-E-acetat, Trolox^{®}, sowie Phenolische Verbindungen und Pflanzenextrakte, diese enthaltend, wie z. B. Flavonoide, z. B. Glycosylrutin, Ferulasäure, Kaffeesäure, Furfurylidenglucitol, Butylhydroxytoluol, Butylhydroxyanisol, Nordihydroguajakharzsäure, Nordihydroguajaretsäure, Trihydroxybutyrophenon und deren Derivate, als Salz-, Ester-, Ether-, Zucker-, Nukleotid-, Nukleosid-, Peptid- und Lipid-Verbidung. Harnsäure und deren Derivate, Mannose und deren Derivate, als Salz-, Ester-, Ether-, Zucker-, Thiol-, Nukleotid-, Nukleosid-, Peptid- und Lipid-Verbidung. Zink und dessen Derivate, z.B. ZnO, ZnSO₄, Selen und dessen Derivate, z.B. Selenmethionin, Ebselen, Stilbene und deren Derivate, z.B. Stilbenoxid, Trans-Stilbenoxid, und die erfindungsgemäß geeigneten Derivate, als Salz-, Ester-, Ether-, Zucker-, Thiol-, Nukleotid-, Nukleosid-, Peptid- und/oder Lipid-Verbidung, dieser genannten Wirkstoffe.

Als Wirkstoffe können desweiteren beispielsweise ätherische Öle eingesetzt werden. Unter ätherischen Ölen sind aus Pflanzen gewonnene Konzentrate zu verstehen, die als natürliche Rohstoffe hauptsächlich in der Parfüm- und Lebensmittelindustrie eingesetzt werden und die mehr oder weniger aus flüchtigen Verbindungen bestehen. Als Beispiele für diese Verbindungen können 1,8-Cineol, Limonen, Menthol, Borneol und Kampfer genannt werden. Oft wird der Begriff ätherische Öle für die noch in den Pflanzen enthaltenen flüchtigen Inhaltsstoffe verwendet. Im eigentlichen Sinn versteht man aber unter ätherischen Ölen Gemische aus flüchtigen Komponenten, die durch Wasserdampfdestillation aus pflanzlichen Rohstoffen hergestellt werden.

Ätherische Öle bestehen ausschließlich aus flüchtigen Komponenten, deren Siedepunkte in der Regel zwischen 150 und 300 °C liegen. Sie enthalten überwiegend Kohlenwasserstoffe oder monofunktionelle Verbindungen wie Aldehyde, Alkohole, Ester, Ether und Ketone. Stammverbindungen sind Mono- und Sesquiterpene, Phenylpropan-Derivate und längerkettige aliphatische Verbindungen.

Bei manchen ätherischen Ölen dominiert ein Inhaltsstoff, zum Beispiel Eugenol in Nelkenöl mit mehr als 85 %, andere ätherische Öle stellen hingegen komplex zusammengesetzte Mischungen der einzelnen Bestandteile dar. Oft werden die organoleptische Eigenschaften nicht von den Hauptkomponenten, sondern von Neben- oder Spurenbestandteilen geprägt, wie zum Beispiel von den 1,3,5-Undecatrienen und Pyrazinen im Galbanum-ÖI. Bei vielen der kommerziell bedeutenden ätherischen Öle geht die Zahl der identifizierten Komponenten in die Hunderte. Sehr viele Inhaltsstoffe sind chiral, wobei sehr oft ein Enantiomer überwiegt oder ausschließlich vorhanden ist, wie zum Beispiel (-)-Menthol im Pfefferminzöl oder (-)-Linalylacetat im Lavendelöl.

Als bevorzugte ätherische Öle können Oleum Eucalypti, Oleum Menthae piperitae, Oleum camphoratum, Oleum Rosmarini, Oleum Thymi, Oleum Pini sibricum und Oleum Pini silverstris sowie die Terpene 1,8-Cineol und Levomethanol genannt werden.

Als weitere ätherische Öle sind Oleum Abietis albae, Oleum Anisi, Oleum Aurantii Floris, Oleum Bergamottae, Oleum Calendulae infusum, Oleum camphoratum, Oleum Caryophylli, Oleum Chamomillae, Oleum Cinnamomi ceylanici, Oleum Citri, Oleum Citronellae, Oleum Cupressi, Oleum Cymbopogonis, Oleum Jecoris, Oleum Lavendulae, Oleum Macidis, Oleum Majoranae, Oleum Melaleucae viridiflorae, Oleum Melissae, Oleum Menthae arvensis, Oleum Menthae piperatae, Oleum Millefolium, Oleum Myrrhae, Oleum Myrte, Oleum Oregani, Oleum Pini sibricum, Oleum Pinisilvestris, Oleum Salviae, Oleum Santali, Oleum Terebinthinae rectificat., Oleum Thymi Oleum Valerianae, Oleum Zingiberis und/oder Teebaumöl zu nennen.

Pfefferminzöle sind durch Wasserdampfdestillation aus Blättern und Blütenständen verschiedener Pfefferminze-Sorten gewonnene ätherische Öle, gelegentlich auch solche aus Mentha arvensis.

Citrusöle sind ätherische Öle, die aus den Schalen von Citrusfrüchten (Bergamotte, Grapefruit, Limette, Mandarine, Orange, Zitrone) gewonnen werden, oft auch Agrumenöle genannt.

Citrusöle bestehen zu einem großen Teil aus Monoterpen-Kohlenwasserstoffen, hauptsächlich Limonen (Ausnahme: Bergamottöl, das nur ca. 40 % enthält).

Beispielsweise kann Menthol zur Oberflächenanästhesierung bei Hautirritationen durch leichte Verbrennungen eingesetzt werden. Die so hergestellten Produkte erzeugen ein angenehmes Kältegefühl und können zur Kühlung von Hautreizungen, z. B. leichter Sonnenbrand und Rasurbrand, die keiner fachärztlichen Behandlung bedürfen, zum Einsatz kommen.

Menthol hat drei asymmetrische C-Atome und kommt demzufolge in vier diastereomeren Enantiomerenpaaren vor. Die Diastereomeren, die destillativ getrennt werden können, werden als Neoisomenthol, Isomenthol, Neomenthol [(+)-Form: Bestandteil des japanischen Pfefferminzöls] und Menthol bezeichnet. Wichtigstes Isomer ist (-)-Menthol (Levomenthol), glänzende, stark pfefferminzartig riechende Prismen.

Als weitere Wirkstoffe kann zum Beispiel Campher zur Behandlung von Hautirritationen/ leichten Schmerzen, Neuralgien und Entzündungen der Matrix zugesetzt werden. Unter Campher versteht man 2-Bornanon, 1,7,7-Trimethylbicyclo[2.2.1]heptan-2-on.

Daneben können für vorteilhafte Ausführungsformen erfindungsgemäßer Hydrogel-Matrixsysteme auch hyperämisierende Wirkstoffe wie natürliche Wirkstoffe des Cayenne-Pfeffers oder synthetische Wirkstoffe wie Nonivamid, Nicotinsäurederivate, bevorzugt Bencylnicotinat oder Propylnicotinat, genannt werden beziehungsweise Antiphlogistika und/oder Analgetika. Beispielhaft seien Capsaicin ([8-Methyl-trans-6-nonensäure-(4-hydroxy-3-methoxybenzylamid)], Nonivamid, Nicotinsäurebenzylester, Benzylnicotinat genannt.

Auch Flavon und seine Derivate, oft auch kollektiv "Flavone" genannt, sind vorteilhafte Zusatzstoffe im Sinne der vorliegenden Erfindung. Sie sind durch folgende Grundstruktur gekennzeichnet (Substitutionspositionen angegeben):

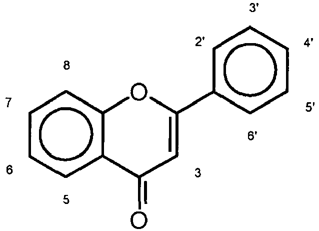

Einige der wichtigeren Flavone, welche auch bevorzugt in erfindungsgemäßen Zubereitungen eingesetzt werden können, sind in der nachstehenden Tabelle aufgeführt:

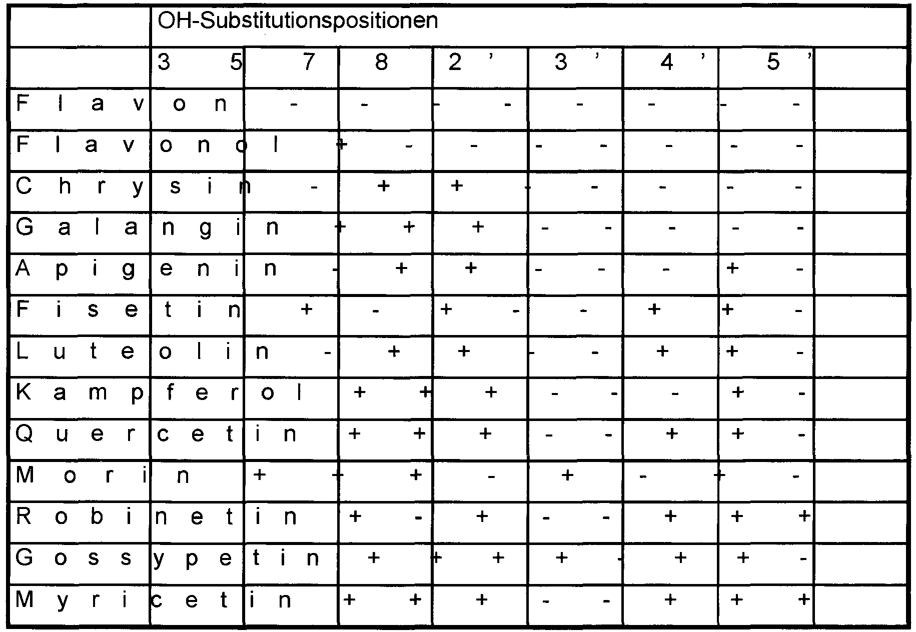

In der Natur kommen Flavone in der Regel in glycosidierter Form vor. Erfindungsgemäß werden die Flavonoide bevorzugt gewählt aus der Gruppe der Substanzen der generischen Strukturformel 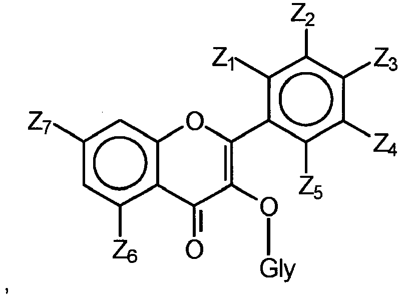 wobei Z₁ bis Z₇ unabhängig voneinander gewählt werden aus der Gruppe H, OH, Alkoxy- sowie Hydroxyalkoxy-, wobei die Alkoxy- bzw. Hydroxyalkoxygruppen verzweigt und unverzweigt sein und 1 bis 18 C-Atome aufweisen können, und wobei Gly gewählt wird aus der Gruppe der Mono- und Oligoglycosidreste.

Erfindungsgemäß können die Flavonoide aber auch vorteilhaft gewählt werden aus der Gruppe der Substanzen der generischen Strukturformel 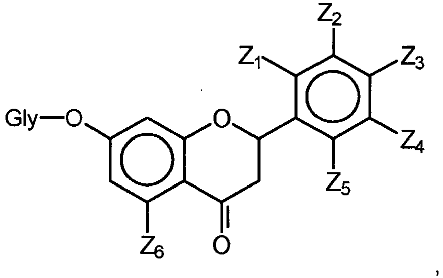 wobei Z₁ bis Z₆ unabhängig voneinander gewählt werden aus der Gruppe H, OH, Alkoxy- sowie Hydroxyalkoxy-, wobei die Alkoxy- bzw. Hydroxyalkoxygruppen verzweigt und unverzweigt sein und 1 bis 18 C-Atome aufweisen können, und wobei Gly gewählt wird aus der Gruppe der Mono- und Oligoglycosidreste.

Bevorzugt können solche Strukturen gewählt werden aus der Gruppe der Substanzen der generischen Strukturformel 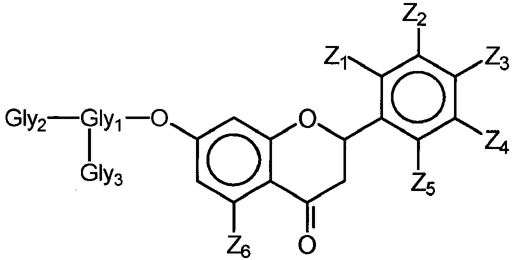 wobei Gly₁, Gly₂ und Gly₃ unabhängig voneinander Monoglycosidreste oder darstellen. Gly₂ bzw. Gly₃ können auch einzeln oder gemeinsam Absättigungen durch Wasserstoffatome darstellen.

Bevorzugt werden Gly₁, Gly₂ und Gly₃ unabhängig voneinander gewählt aus der Gruppe der Hexosylreste, insbesondere der Rhamnosylreste und Glucosylreste. Aber auch andere Hexosylreste, beispielsweise Allosyl, Altrosyl, Galactosyl, Gulosyl, Idosyl, Mannosyl und Talosyl sind gegebenenfalls vorteilhaft zu verwenden. Es kann auch erfindungsgemäß vorteilhaft sein, Pentosylreste zu verwenden.

Vorteilhaft werden Z₁ bis Z₅ unabhängig voneinander gewählt aus der Gruppe H, OH, Methoxy-, Ethoxy- sowie 2-Hydroxyethoxy-, und die Flavonglycoside haben die Struktur

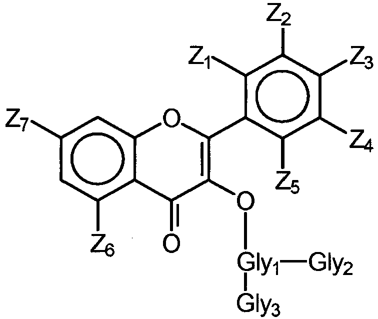

Besonders vorteilhaft werden die erfindungsgemäßen Flavonglycoside aus der Gruppe, welche durch die folgende Struktur wiedergegeben werden: 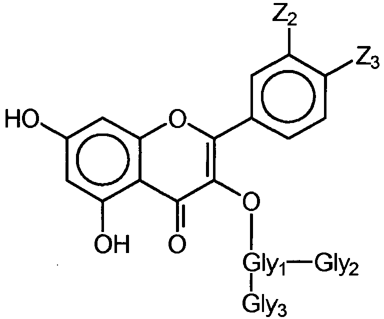 wobei Gly₁, Gly₂ und Gly₃ unabhängig voneinander Monoglycosidreste oder Oligoglycosidreste darstellen. Gly₂ bzw. Gly₃ können auch einzeln oder gemeinsam Absättigungen durch Wasserstoffatome darstellen.

Bevorzugt werden Gly₁, Gly₂ und Gly₃ unabhängig voneinander gewählt aus der Gruppe der Hexosylreste, insbesondere der Rhamnosylreste und Glucosylreste. Aber auch andere Hexosylreste, beispielsweise Allosyl, Altrosyl, Galactosyl, Gulosyl, Idosyl, Mannosyl und Talosyl sind gegebenenfalls vorteilhaft zu verwenden. Es kann auch erfindungsgemäß vorteilhaft sein, Pentosylreste zu verwenden.

Besonders vorteilhaft im Sinne der vorliegenden Erfindung ist, das oder die Flavonglycoside zu wählen aus der Gruppe α-Glucosylrutin, α-Glucosylmyricetin, α-Glucosylisoquercitrin, α-Glucosylisoquercetin und α-Glucosylquercitrin.

Erfindungsgemäß besonders bevorzugt ist α-Glucosylrutin.

Erfindungsgemäß vorteilhaft sind auch Naringin (Aurantiin, Naringenin-7-rhamnoglucosid), Hesperidin (3',5,7-Trihydroxy-4'-methoxyflavanon-7-rutinosid, Hesperidosid, Hesperetin-7-O-rutinosid). Rutin (3,3',4',5,7-Pentahydroxyflyvon-3-rutinosid, Quercetin-3-rutinosid, Sophorin, Birutan, Rutabion, Taurutin, Phytomelin, Melin), Troxerutin (3,5-Dihydroxy-3',4',7-tris(2-hydroxyethoxy)-flavon-3-(6-O-(6-deoxy-α-L-mannopyranosyl)-β-D-glucopyranosid)), Monoxerutin (3,3',4',5-Tetrahydroxy-7-(2-hydroxyethoxy)-flavon-3-(6-O-(6-deoxy-α-L-mannopy-ranosyl)-β-D-glucopyranosid)), Dihydrorobinetin (3,3',4',5',7-Pentahydroxyflavanon), Taxifolin (3,3',4',5,7-Pentahydroxyflavanon), Eriodictyol-7-glucosid (3',4',5,7-Tetrahydroxyflavanon-7-glucosid), Flavanomareïn (3',4',7,8-Tetrahydroxyflavanon-7-glucosid) und Isoquercetin (3,3',4',5,7-Pentahydroxyflavanon-3-(β-D-Glucopyranosid) oder deren Derivate.

Bevorzugter Wirkstoff ist ebenfalls n-4-Butylresorcinol.

Weitere bevorzugte Wirkstoffklassen einer erfindungsgemäßen Gelmatrix sind - ohne den Anspruch der Vollständigkeit im Rahmen der vorliegenden Erfindung zu erheben:
Antimykotika, wie z. B. Nafitin, Amorrolfin, Tolnaftat, Ciclopirox; Nichtsteroidale Antirheumatika, wie z. B. Glykolsalicylat, Flufenaminsäure, Ibuprofen, Etofenamat, Ketoprofen, Piroxicam, Indomethacin; Antiphlogistika, wie Acetylsalicylsäure; Antipuriginosa, wie z. B. Polidocanol, Isoprenalin, Crotamiton; Lokalanästhetika, wie z. B. Lidocain, Benzocain
Antipsoriatika, wie z. B. Ammoniumbitumasulfonat; Keratolytika, wie z.B. Harnstoff.

Von gesonderter Bedeutung unter den Wirkstoffen sind für erfindungsgemäße Hydrogel-Matrixsysteme die Desinfektionsmittel beziehungsweise Antiseptika hervorzuheben. Antiseptika sind besonders geeignet zur Behandlung der Haut.

Besonders geeignet als Antiseptikum ist Chlorhexidin, internationaler Freiname für 1,1'-Hexamethylenbis[5-(4-chlorphenyl)-biguanid], wobei als Antiseptikum das Dihydrochlorid, Diacetat und Digluconat verwendet werden, sowie auch Octenidin, 1,1'-(1,10-Decandiyl)bis[4-(octylamino)pyridinium]-dichlorid und Polihexanid, Polyaminopropyl-biguanid.

### Ausgestaltung / Anwendungsform

Zur Anwendung als Pflaster bzw. kosmetische Matrix / kosmetisches Pad werden die erfindungsgemäßen Hydrogel-Matrixsysteme als Schicht auf ein Trennmedium aus Papier, Folie o. ä. gepresst, gewalzt o. ä. und auf der Rückseite mit einem beliebigen Trägermaterial wie z.B. einer Polymerfolie, Textilien o.ä. kaschiert. Erfindungsgemäß besonders bevorzugt werden die Gelmatrices im warmen, flüssigen Zustand mittels Dosierpumpe auf ein Trägermaterial aufgetragen und ganz besonders bevorzugt durch entsprechende Kavitäten in den Press- oder Walzwerken in einer dreidimensionalen Form ausgeführt. Die Form der erzeugten Pflaster bzw. kosmetischen Matrix wird durch die Form der Kavitäten bestimmt und unterliegt keiner Einschränkung, sie kann z. B. ellipsoid mit flach auslaufenden Rändern oder beispielsweise eckig ausgeführt sein.

Besonders vorteilhaft ist die erfindungsgemäße Hydrogel-Matrix auf einer flexiblen Deckschicht aufgebracht, insbesondere bei der Verwendung als Pflaster oder kosmetische Patch. Aufgebaut ist ein entsprechendes Pflaster bzw. kosmetisches Patch aus einem Träger wie Folien, Vliese, Gewebe, Schäume etc., der Klebmatrix und Abdeckfolie, Abdeckpapier oder Trennpapier zum Schutz der klebenden Matrix vor dem Gebrauch des Pflasters bzw. kosmetischen Patches.

In einer weiteren bevorzugten Ausführungsform der Erfindung werden als Träger Polymerfolien, Vliese, Gewebe sowie deren Kombinationen eingesetzt. Als Trägermaterialien stehen u. a. Polymere wie Polyethylen, Polypropylen, Polyester, Polyether, Polyether-ester Copolymere und Polyurethan oder auch Naturfasern zur Auswahl.

Zusammenfassend kann festgehalten werden, dass als Trägermaterialien sich alle starren und elastischen Flächengebilde aus synthetischen und natürlichen Rohstoffen eignen. Bevorzugt sind Trägermaterialien, die so eingesetzt werden können, dass sie Eigenschaften eines funktionsgerechten Verbandes erfüllen. Beispielhaft sind Textilien wie Gewebe, Gewirke, Gelege, Vliese, Laminate, Netze, Folien, Schäume und Papiere aufgeführt. Weiter können diese Materialien vor- beziehungsweise nachbehandelt werden. Gängige Vorbehandlungen sind Corona und Hydrophobieren; geläufige Nachbehandlungen sind Kalandern, Tempern, Kaschieren, Stanzen und Eindecken.

Besonders vorteilhaft ist, wenn das Trägermaterial sterilisierbar, bevorzugt γ-(gamma) sterilisierbar, ist.

Erfindungsgemäß ganz besonders bevorzugt sind Trägermaterialien mit einer guten Sauerstoff-, Luft- und Wasserdampfdurchlässigkeit, welche im Siebdruck oder analogen Verfahren punktuell mit stark klebenden Polymeren wie Polyisobutylen, SEBS-Blockpolymeren, Natur- und/oder Synthesekautschuken, Polyurethan o. ä. versehen sind und an den Seitenrändern die aufgebrachte Hydrogelmatrix nach außen überlappen. Dergestalt ausgefertigte erfindungsgemäße Matrices können an mechanisch stark beanspruchten Körperteilen wie Ellenbogen oder Kniegelenken selbstklebend fixiert werden, wo das eigene Haftvermögen der Hydrogel-Matrixsysteme für eine dauerhafte Applikation nicht mehr genügt.

Die genannten Eigenschaften der Klebmatrix legen insbesondere die Verwendung für medizinische Produkte, insbesondere Pflaster, medizinische Fixierungen, Wundabdeckungen, orthopädische oder phlebologische Bandagen und Binden nahe. Ganz besonders bevorzugt ist die Verwendung als kosmetisches oder dermatologisches Pad (Patch).

Schließlich kann die Gelmatrix mit einem klebstoffabweisenden Trennträgermaterial, wie silikonisiertes Papier, eingedeckt oder mit einer Wundauflage oder einer Polsterung versehen werden. Auf seiner selbstklebend ausgerüsteten, später der Haut zugewandten Seite ist die erfindungsgemäße kosmetische Matrix über seine ganze Breite bis zum Gebrauch üblicherweise mit einem klebstoffabweisenden Trennträgermaterial abgedeckt. Dieses schützt die Selbstklebeschicht aus der gut hautverträglichen Klebemasse der Gelmatrix, die vorzugsweise im Transferverfahren aufgebracht worden ist, und stabilisiert zusätzlich das ganze Produkt. Die Abdeckung kann in bekannter Weise einstückig oder vorzugsweise zweiteilig ausgebildet sein.

Weitere Ausführungsformen können dergestalt sein, dass zwischen der Rückseite der Matrix und dem Abdeckträger sich eine zweite Matrix mit höherer Wirkstofflöslichkeit als Reservoir befindet. Dies könnte statt einer zweiten Matrix und Träger auch eine Tiefziehfolie mit reinem Wirkstoff sein.

Auf der Klebseite der Matrix befindet sich teilweise, z. B. am Rand, eine zweite Matrix mit hoher Klebkraft zur zusätzlichen Fixierung, aber ungenügender Wirkstofflöslichkeit.

Die wirkstofffreie Matrix befindet sich zwischen zwei nicht verankernden Folien und wird zur Fixierung genutzt.

Gegenstand der vorliegenden Erfindung ist ferner die Verwendung kosmetischer oder dermatologischer Wirkstoffe in den erfindungsgemäßen Gelmatrices.

Insbesondere die Verwendung der wirkstoffdotierten Gelmatrices auf Polyacrylsäure-Agar-Agar-Basis zur Anwendung als Pads zur kosmetischen und wohltuenden Behandlung von unerwünschten Hauterscheinungen ist bevorzugt hervorzuheben. Insbesondere bei der Behandlung von Hautalterungserscheinungen, insbesondere Hautfalten, bei Pigmentierungsstörungen, insbesondere von Altersflecken, und entzündlichen/irritativen Hauterscheinungen, beispielsweisebei Rasurbrand und/oder Sonnenbrand.

Es ist bevorzugt im Sinne der vorliegenden Erfindung, wenn die erfindungsgemäßen kosmetischen oder dermatologischen Matrices zusätzlich einen oder mehrere Alkohole enthalten, insbesondere, wenn die Formulierungen in Form eines Aftersun-Präparats vorliegen und sich durch eine besondere Kühlwirkung auszeichnen sollen.

Die Verwendung der erfindungsgemäßen Hydrogel-Matrix als kosmetische oder dermatologische Pads oder Pflaster ist besonders in flächiger Ausführungsform mit einer Gesamtfläche von 0,2 bis 1000 cm² geeignet. Damit werden beispielweise kleine (0,2 - 2 cm²) Bereiche der Haut oder großflächige Bereiche (bis zu 1000 cm²) zur intensiven Kühlung abgedeckt.

Bevorzugt ist die Verwendung der erfindungsgemäßen, selbstklebenden Hydrogel-Matrix in flächiger oder räumlicher Ausführungsform mit einem Polymermatrixgewichtsanteil von 0,1 bis 1000 g, insbesondere von 500 g. Die Form kann dabei rund, oval, eckig oder den Hautpartien angepasst gestaltet sein.

### Beschreibung der Klebkraftmessung

Zunächst werden zwei Stahlplatten für die Messungen vorbereitet. Gelagert werden Proben und Platten in einem Klimaraum, in dem auch die Messungen vorgenommen werden. Die konstanten Raumbedingungen liegen bei 23 ± 2 °C und 50 ± 6 % relative Luftfeuchtigkeit.

Die für den jeweiligen Tag der Messungen benötigten Proben werden einzeln in Aluminiumfolie licht- und luftdicht verpackt und im Klimaraum gelagert. Für die Durchführung der Messungen wird immer ein Beutel geöffnet und die Pflastermatrix innerhalb von 30 Minuten nach Öffnung vorbereitet und gemessen.

Wenn der Teststreifen vom Trennpapier gelöst, mittig auf die Stahlplatte gelegt und von Hand leicht festgestrichen ist, wird die Platte mit der Probe in eine Vorrichtung zum Anrollen gelegt.

Diese Vorrichtung besteht aus einer Rolle mit 80 mm Durchmesser und einem Gewicht von 3000 g. Um eine Anrollkraft von 20 N/cm Probenbreite zu erreichen werden zwei zusätzliche Gewichte an der Rolle angebracht. Diese Rolle fährt nun - bei einer Geschwindigkeit von 10 m/s - 10 Mal vor und zurück über die Stahlplatte mit dem Teststreifen. Die Anwendung einer solchen Vorrichtung stellt sicher, dass jede Probe auf genau gleiche Art und Weise auf der Stahlplatte fixiert wird und sorgt für vergleichbare Messergebnisse.

Zur Messung wird die Stahlplatte zwischen den pneumatischen Klemmen eines Zwick-Geräts eingespannt. Ein Streifen Tesaband (Typ 4651) wird am Messkopf befestigt. Er ist so lang gewählt, dass am unteren Ende Teststreifen und Tesastreifen zusammengeklebt werden können.

Verwendet wird der Messkopf vom Typ KAP-Z mit einem Messbereich von 0,04 bis 10 N/cm. Er besitzt bei Messwerten von 0,04 bis 0,2 N/cm die Ungenauigkeitsklasse 1.

Das Gerät fährt nun den Messkopf nach oben, zieht somit den Teststreifen von der Stahlplatte ab, zeichnet kontinuierlich am Computer die dabei benötigte Kraft über die Länge des Messweges auf und errechnet aus den aufgezeichneten Daten den Mittelwert als finale Klebkraftangabe.

### Messung der Oberflächenenergie

Die Oberflächenenergie bzw. die Oberflächenspannung der Muster wird durch eine Kontaktwinkelmessung bestimmt. Dabei werden 3 Testflüssigkeiten mit bekannter Oberflächenenergie auf die zu messende Oberfläche getropft und der Winkel den die Tropfen zur Oberfläche bilden in eine Oberflächenenergie umgerechnet.

Der Kontaktwinkel wird bestimmt, indem eine Software um den Tropfen auf der Oberfläche des Substrats eine Ellipse bzw. einen Kreis berechnet. An diese Ellipse (oder Kreis) wird am Schnittpunkt mit der Oberfläche des Substrats auf beiden Seiten des Tropfens eine Tangente angelegt. Diese Tangente bildet dann mit der Oberfläche des Substrats den Winkel, der an beiden Seiten abgelesen wird. Beispiel:
Gemessene Kontaktwinkel für Basisrezeptur 2 mit 2,5 Gew.-% Myrj 59P:
   Wasser 55,38° ± 1,69°
   Glycerin 92,97° ± 1,66°
   Dijodmethan 38,00° ± 7,92°
   daraus errechnete Oberflächenenergie: 25,58 mN/m

Die dabei verwendete Berechnungsmethodik, die auch die oben aufgeführten Kontaktwinkel für die Testflüssigkeiten auf einem erfindungsgemäßen Substrat einbezieht, folgt dabei der für niederenergetische Oberflächen häufig angewendeten Methodik nach Owens, Wendt, Rabel und Kaelble (OWRK). Die Messung und Berechnung der Oberflächenenergie ist in der Literatur beschrieben, z.B. http://www.kruss.de/theorie/messungen/kontaktwinkel/einfuehrung.html, http://www.kruss.de/theorie/messungen/kontaktwinkel/modelle/owrk.html

## Patentansprüche

1. Selbstklebendes Hydrogel-Matrixsystem enthaltend Wasser, mindestens eine Basiskomponente, mindestens einen Strukturbildner und mindestens ein nichtionisches Tensid, **dadurch gekennzeichnet, dass** das nichtionische Tensid einen HLB-Wert ≥ 14, insbesondere ≥ 16 aufweist und das Hydrokolloid-Matrixsystem eine Oberflächenenergie zwischen 25 und 40 mN/m, insbesondere zwischen 25 und 30 mN/m aufweist.

2. Selbstklebende Hydrogel-Matrix nach Anspruch 1 geeignet für Transdermale Pflaster, **dadurch gekennzeichnet, dass** es mindestens einen Wirkstoff aufweist und die Klebkraft auf Stahl der Matrix mindestens 0,3 N/cm beträgt.

3. Selbstklebende Hydrogel-Matrix nach Anspruch 2, **dadurch gekennzeichnet, dass** der Wirkstoff oder mindestens ein Wirkstoff gewählt werden aus der Gruppe der kosmetischen, dermatologische und/oder pharmazeutische Wirkstoffe.

4. Selbstklebende Hydrogel-Matrix nach Anspruch 3, **dadurch gekennzeichnet, dass** der Wirkstoff gewählt wird aus der Gruppe n-4-Butyl-Resorcinol, Paeonol, Magnolien-Extrakt, Magnolol, Honokiol, Q10, AGR, Glycerylglucose, Ibuprofen und dessen Salze, Etofenamat, Indometacin, Diclofenac und dessen Salze, Acetylsalicylsäure, Licochalcone A, Bakuchiol, Aciclovir, Decandiol, Carnitin, Rucinol und/oder Salicylsäure.

5. Selbstklebende Hydrogel-Matrix nach Anspruch 2, **dadurch gekennzeichnet, dass** der Wirkstoff oder mindestens ein Wirkstoff ein desinfizierender und/oder keimhemmender Wirkstoff ist.

6. Verwendung einer selbstklebenden Hydrogel-Matrix aufweisend Wasser, mindestens eine Basiskomponente, mindestens einen Strukturbildner und mindestens ein nichtionisches Tensid zur Herstellung eines Pads oder Pflasters zur Behandlung von unerwünschten Hauterscheinungen, insbesondere zur Behandlung von Hautalterungserscheinungen (ganz besonders zur Behandlung von Hautfalten), Pigmentierungsstörungen (ganz besonders zur Behandlung von Altersflecken), und entzündlichen Hauterscheinungen, irritativen Hauterscheinungen (ganz besonders zur Behandlung von Rasurbrand und/oder Sonnenbrand), **dadurch gekennzeichnet, dass** das nichtionische Tensid einen HLB-Wert ≥ 14, insbesondere ≥ 16 aufweist und das Hydrokolloid-Matrixsystem eine Oberflächenenergie zwischen 25 und 40 mN/m, insbesondere zwischen 25 und 30 mN/m aufweist.

7. Verwendung einer selbstklebenden Hydrogel-Matrix aufweisend Wasser, mindestens eine Basiskomponente, mindestens einen Strukturbildner und mindestens ein nichtionisches Tensid zur Herstellung eines Pads oder Pflasters zur intensiven Kühlung der Haut, **dadurch gekennzeichnet, dass** das nichtionische Tensid einen HLB-Wert ≥ 14, insbesondere ≥ 16 aufweist und das Hydrokolloid-Matrixsystem eine Oberflächenenergie zwischen 25 und 40 mN/m, insbesondere zwischen 25 und 30 mN/m aufweist.

8. Verwendung einer selbstklebenden Hydrogel-Matrix aufweisend Wasser, mindestens eine Basiskomponente, mindestens einen Strukturbildner und mindestens ein nichtionisches Tensid zur Herstellung eines Aftersun-Präparates, **dadurch gekennzeichnet, dass** das nichtionische Tensid einen HLB-Wert ≥ 14, insbesondere ≥ 16 aufweist und das Hydrokolloid-Matrixsystem eine Oberflächenenergie zwischen 25 und 40 mN/m, insbesondere zwischen 25 und 30 m N/m aufweist.

## Claims

1. Self-adhesive hydrogel matrix system comprising water, at least one base component, at least one structure former and at least one nonionic surfactant, **characterized in that** the nonionic surfactant has a HLB value ≥ 14, in particular ≥ 16, and the hydrocolloid matrix system has a surface energy between 25 and 40 mN/m, in particular between 25 and 30 mN/m.

2. Self-adhesive hydrogel matrix according to Claim 1 suitable for transdermal patches, **characterized in that** it has at least one active ingredient and the adhesive strength to steel of the matrix is at least 0.3 N/cm.

3. Self-adhesive hydrogel matrix according to Claim 2, **characterized in that** the active ingredient or at least one active ingredient are selected from the group of cosmetic, dermatological and/or pharmaceutical active ingredients.

4. Self-adhesive hydrogel matrix according to Claim 3, **characterized in that** the active ingredient is selected from the group n-4-butyl-resorcinol, paeonol, magnolia extract, magnolol, honokiol, Q10, AGR, glycerylglucose, ibuprofen and salts thereof, etofenamate, indometacin, diclofenac and salts thereof, acetylsalicylic acid, licochalcone A, bakuchiol, acyclovir, decanediol, carnitine, rucinol and/or salicylic acid.

5. Self-adhesive hydrogel matrix according to Claim 2, **characterized in that** the active ingredient or at least one active ingredient is a disinfectant and/or germicidal active ingredient.

6. Use of a self-adhesive hydrogel matrix having water, at least one base component, at least one structure former and at least one nonionic surfactant for producing a pad or patch for treating undesired skin symptoms, in particular for treating skin aging symptoms (very particularly for treating skin wrinkles), pigmentation disorders (very particularly for treating age spots), and inflammatory skin symptoms, irritative skin symptoms (very particularly for treating razor burn and/or sunburn), **characterized in that** the nonionic surfactant has a HLB value ≥ 14, in particular ≥ 16, and the hydrocolloid matrix system has a surface energy between 25 and 40 mN/m, in particular between 25 and 30 mN/m.

7. Use of a self-adhesive hydrogel matrix having water, at least one base component, at least one structure former and at least one nonionic surfactant for producing a pad or patch for the intensive cooling of the skin, **characterized in that** the nonionic surfactant has a HLB value ≥ 14, in particular 16, and the hydrocolloid matrix system has a surface energy between 25 and 40 mN/m, in particular between 25 and 30 mN/m.

8. Use of a self-adhesive hydrogel matrix having water, at least one base component, at least one structure former and at least one nonionic surfactant for producing an aftersun preparation, **characterized in that** the nonionic surfactant has a HLB value ≥ 14, in particular ≥ 16, and the hydrocolloid matrix system has a surface energy between 25 and 40 mN/m, in particular between 25 and 30 mN/m.

## Revendications

1. Système de matrice à hydrogel autoadhésif, contenant de l'eau, au moins un composant de base, au moins un agent structurant et au moins un tensioactif non ionique, **caractérisé en ce que** le tensioactif non ionique présente une valeur HLB ≥ 14, en particulier ≥ 16 et le système de matrice-hydrocolloïde présente une énergie superficielle comprise entre 25 et 40 mN/m, en particulier entre 25 et 30 mN/m.

2. Matrice à hydrogel autoadhésive selon la revendication 1, appropriée à des timbres transdermiques, **caractérisée en ce qu'**elle comporte au moins une substance active et le pouvoir adhésif de la matrice sur acier est d'au moins 0,3 N/cm.

3. Matrice à hydrogel autoadhésive selon la revendication 2, **caractérisée en ce qu'**on choisit la substance active ou au moins une substance active dans le groupe des substances actives cosmétiques, dermatologiques et/ou pharmaceutiques.

4. Matrice à hydrogel autoadhésive selon la revendication 3, **caractérisée en ce que** la substance active est choisie dans le groupe constitué par le n-4-butyl-résorcinol, la paeonol, l'extrait de magnolia, le magnolol, l'honokiol, Q10, AGR, le glycérylglucose, l'ibuprofène et ses sels, l'étofénamate, l'indométacine, le dicofénac et ses sels, l'acide acétylsalicylique, la licochalcone A, le bakuchiol, l'aciclovir, le décanediol, la carnitine, le rucinol et/ou l'acide salicylique.

5. Matrice à hydrogel autoadhésive selon la revendication 2, **caractérisée en ce que** la substance active ou au moins une substance active est une substance active désinfectante et/ou antiseptique.

6. Utilisation d'une matrice à hydrogel autoadhésive, comportant de l'eau, au moins un composant de base, au moins un agent structurant et au moins un tensioactif non ionique, pour la fabrication d'un coussinet ou d'un emplâtre pour le traitement de manifestations cutanées indésirables, en particulier pour le traitement de manifestations de vieillissement de la peau (tout particulièrement pour le traitement de plis de la peau), de troubles de la pigmentation (tout particulièrement pour le traitement de taches de sénescence), et de manifestations cutanées inflammatoires, de manifestations d'irritation de la peau (tout particulièrement pour le traitement du feu du rasoir et/ou de coup de soleil), **caractérisée en ce que** le tensioactif non ionique présente une valeur HLB ≥ 14, en particulier ≥ 16 et le système de matrice-hydrocolloïde présente une énergie superficielle comprise entre 25 et 40 mN/m, en particulier entre 25 et 30 nM/m.

7. Utilisation d'une matrice à hydrogel autoadhésive, comportant de l'eau, au moins un composant de base, au moins un agent structurant et au moins un tensioactif non ionique, pour la fabrication d'un coussinet ou d'un emplâtre destiné au refroidissement intensif de la peau, **caractérisée en ce que** le tensioactif non ionique présente une valeur HLB ≥ 14, en particulier ≥ 16 et le système de matrice-hydrocolloïde présente une énergie superficielle comprise entre 25 et 40 mN/m, en particulier entre 25 et 3D nM/m.

8. Utilisation d'une matrice à hydrogel autoadhésive, comportant de l'eau, au moins un composant de base, au moins un agent structurant et au moins un tensioactif non ionique, pour la fabrication d'une préparation après-soleil, **caractérisée en ce que** le tensioactif non ionique présente une valeur HLB ≥ 14, en particulier ≥ 16 et le système de matrice-hydrocolloïde présente une énergie superficielle comprise entre 25 et 40 mN/m, en particulier entre 25 et 30 nM/m.
